(19) Europäisches Patentamt
European Patent Office
Office européen des brevets

(11) EP 3 097 109 B1

(12) **FASCICULE DE BREVET EUROPEEN**

(45) Date de publication et mention de la délivrance du brevet:
**02.10.2019 Bulletin 2019/40**

(21) Numéro de dépôt: **15704349.8**

(22) Date de dépôt: **21.01.2015**

(51) Int Cl.:
*C07D 493/04* (2006.01)

(86) Numéro de dépôt international:
**PCT/FR2015/050148**

(87) Numéro de publication internationale:
**WO 2015/110758 (30.07.2015 Gazette 2015/30)**

(54) **PROCEDE DE FABRICATION DE GLYCIDYL ETHERS D'ISOHEXIDES**

VERFAHREN ZUR HERSTELLUNG VON ISOHEXIDGLYCIDYLETHERN

PROCESS FOR PRODUCING ISOHEXIDE GLYCIDYL ETHERS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.01.2014 FR 1450474**

(43) Date de publication de la demande:
**30.11.2016 Bulletin 2016/48**

(73) Titulaire: **Roquette Frères**
**62136 Lestrem (FR)**

(72) Inventeurs:
- **BUFFE, Clothilde**
  **F-59160 Lomme (FR)**
- **DOLENEC, Amélie**
  **F-62660 Beuvry (FR)**
- **IBERT, Mathias**
  **F-59930La Chapelle d'Armentieres (FR)**

(74) Mandataire: **Cabinet Plasseraud**
**66, rue de la Chaussée d'Antin**
**75440 Paris Cedex 09 (FR)**

(56) Documents cités:
**EP-A1- 2 301 941 WO-A1-2008/147473**

- **DAVY-LOUIS VERSACE ET AL: "A Tris(triphenylphosphine)ruthenium(II) Complex as a UV Photoinitiator for Free-Radical Polymerization and in Situ Silver Nanoparticle Formation in Cationic Films", MACROMOLECULES, vol. 46, no. 22, 26 novembre 2013 (2013-11-26), pages 8808-8815, XP055112129, ISSN: 0024-9297, DOI: 10.1021/ma4019872**
- **CHATTI S ET AL: "Cation and leaving group effects in isosorbide alkylation under microwave in phase transfer catalysis", TETRAHEDRON, ELSEVIER SCIENCE PUBLISHERS, AMSTERDAM, NL, vol. 57, no. 20, 14 mai 2001 (2001-05-14), pages 4365-4370, XP004239462, ISSN: 0040-4020, DOI: 10.1016/S0040-4020(01)00318-0**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour principal objet un procédé de fabrication d'une composition d'éthers de bis-anhydrohexitols et notamment de glycidyl éthers d'isohexides, dont une des originalités repose sur une distillation azéotropique conduite sous pression réduite. De telles compositions, produits sont utilisés pour fabriquer des résines époxy, leur fonction étant de former un réseau macromoléculaire tridimensionnel. Les compositions obtenues selon le procédé objet de l'invention sont riches en dérivés diépoxy d'isosorbide au détriment des dérivés monoépoxy, seuls les premiers participant à la formation du réseau tridimensionnel. On augmente donc la densité de réticulation, ce qui permet d'obtenir un matériau plus résistant chimiquement et mécaniquement et présentant une température de transition vitreuse (Tg) plus élevée, par rapport aux mêmes matériaux obtenus avec les éthers de bis-anhydrohexitols selon l'art antérieur.

Le glycidyl éther de Bisphenol A (BADGE ou DGEBA), de formule (I), est un composé chimique utilisé comme agent réticulant dans la fabrication des résines epoxy. Ce produit figure aujourd'hui sur la liste des cancérogènes du groupe 3 du CIRC (Centre International de Recherche sur le Cancer), c'est-à-dire qu'il s'agit d'une substance inclassable quant à sa cancérogénicité pour l'homme.

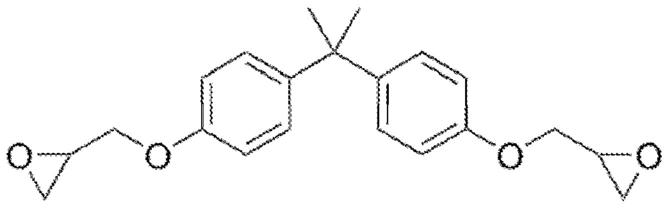

(I)

Or, le DGEBA est notamment utilisé comme additif dans les revêtements pour certaines boîtes de conserve. Du DGEBA libre peut donc se retrouver dans le contenu de ces boîtes, ce qui alimente de nombreuses questions au sujet de sa cancérogénicité (« Détermination of Bisphenol A diglycidyl ether and its hydrolysis products in canned oily foods from the Austrian market », Z. Lebensm. Unters. Forsch. A 208 (1999) pp. 208-211).

Il est connu depuis quelques années que ce produit peut être remplacé par du glycidyléther d'isosorbide, dont la structure est représentée ci-dessous. (formule (II)). Cette structure mime de très près celle du DGEBA.

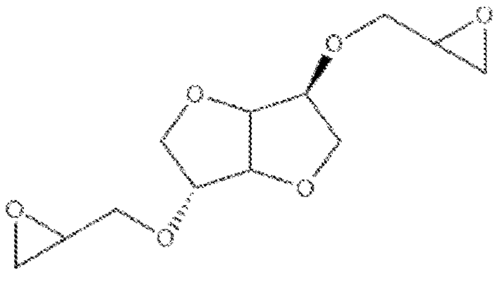

(II)

Ces composés, qui appartiennent à la classe plus générale des éthers de bis-anhydrohexitols, sont aujourd'hui largement connus et décrits dans la littérature, de même que leur procédé de synthèse.

Un des procédés de synthèse connu repose sur la formation initiale d'une solution d'un sel d'isohexide en présence d'espèces très réactives et souvent dangereuses, tels que l'hydrure de sodium ou le sodium métallique, puis sur la réaction avec de l'épichlorhydrine. Le document US 3,272,845 en est une illustration.

Le document US 4,770,871 propose une méthode alternative, qui évite de faire appel à l'hydrure métallique ou au sodium métallique. Cette méthode consiste à faire réagir un bis-anhydrohexitol et un carbonate d'alkyle en présence d'un catalyseur basique, et dans des conditions de température et de pression élevées (200 à 300°C, 5 MPa).

On connaît aussi le document WO 2008 147472 qui décrit un autre procédé ne mettant pas en oeuvre les composés dangereux précités. Ce document propose une méthode de synthèse par mise en solution d'un isohexitol dans un solvant, ajout d'une base, conduite d'une distillation azéotropique avec le solvant, ajout d'un composé choisi dans le groupe consistant en un halogénure d'alkyle ou d'aralkyle, et un ester sulfonate de l'alcool équivalent à l'alcoxyde, et chauffage pour réaliser la réaction d'étherification et obtenir le produit recherché. Les solvants mis en oeuvre sont des solvants aromatiques, comme le toluène et le benzène. Ces solvants s'accommodent mal des réglementations

actuelles qui visent à limiter l'emploi de ces composés souvent nocifs et parfois même toxiques.

Le document US 3,041,300 propose quant à lui une méthode qui ne fait pas appel à des solvants, ni à l'hydrure métallique ou au sodium métallique. Le procédé en question consiste à faire réagir à pression atmosphérique et à chaud (environ 110°C) l'isosorbide et l'épichlorhydrine, à ajouter très lentement un réactif basique comme une solution de soude (pendant au moins 4 heures), et à réaliser une distillation azéotropique. Après filtration et rinçage, on récupère alors l'éther de bis-anhydrohexitol ainsi formé. Plus récemment, le document WO 2012 157832 a proposé une variante de cette technique, en réalisant cette fois la réaction entre l'isosorbide et l'épichlorhydrine toujours sous pression atmosphérique mais à une température plus modérée (40°C).

Enfin, le document WO 2008 147473 décrit 3 des procédés précédents :

- dans son exemple 1, la méthode basée sur une distillation azéotropique en présence de solvant selon le document WO 2008 147472,
- dans son exemple 2, la méthode utilisant de l'hydrure de sodium selon le document US 3,272,845,
- dans son exemple 4, la méthode reposant sur l'ajout très lent de soude et la distillation azéotropique selon les documents US 3,041,300 et WO 2012 157832.

Ce document WO 2008 147473 enseigne une autre route qui est un procédé en 2 étapes, la première consistant à faire réagir l'isohexitol avec l'épichlorhydrine en présence de trifluorure de bore, puis à ajouter une solution alcaline (exemple 3 de ce document). Néanmoins, on sait que le trifluorure de bore est un gaz toxique et incolore qui réagit avec l'air humide en formant des fumées blanches composées de fluorure d'hydrogène, d'acide borique et d'acide fluoroborique.

Lorsqu'on met en oeuvre l'un quelconque des procédés décrits précédemment, on obtient en fait une composition (par opposition à un produit pur) contenant notamment des dérivés mono et difonctionnels d'éthers de bis-anhydrohexitols. Or, seuls ces derniers participent au réseau macromoléculaire tridimensionnel au cours de la fabrication de la résine, notamment en présence de durcisseurs type amines. Ce sont donc ces dérivés difonctionnels qu'on cherchera à privilégier au détriment des produits monofonctionnels.

De la même manière, on veillera à limiter la teneur en oligomère (III) afin d'obtenir un réseau tridimensionnel ayant une densité de réticulation plus élevée. En effet, plus n est grand, plus la distance entre 2 fonctions réactives est grande et donc plus la distance entre chaque noeud de réticulation sera grande. Une densité de noeud importante permet d'obtenir un matériau ayant une température de transition vitreuse (Tg) plus élevée et plus résistant chimiquement et mécaniquement.

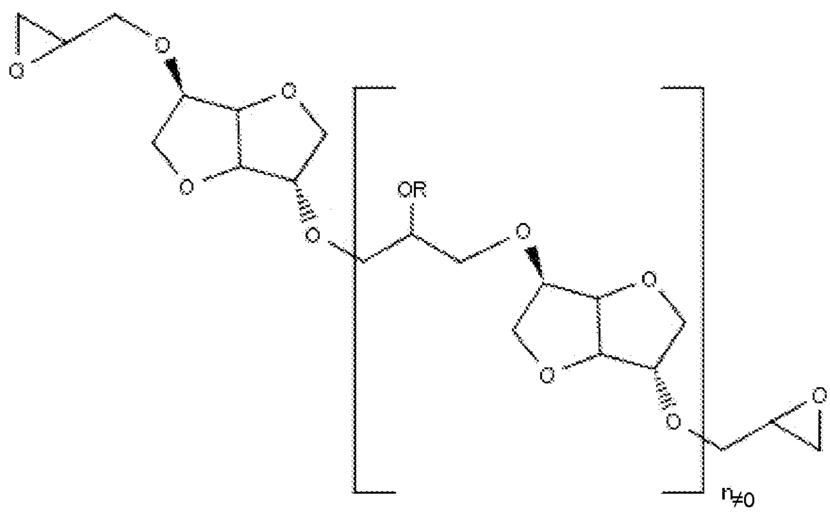

R=H ou $CH_2$-$(CH)_2$-O

(III)

La présence d'oligomères et/ou de mono-glycidyléthers d'isosorbide peut être directement reliée à l'équivalent d'époxy en poids, défini comme la masse de résine contenant un équivalent de fonction glycidyle. Par exemple, le diglycidyléther d'isosorbide (figure II) qui a un poids moléculaire de 258 g / mol et qui contient 2 fonctions glycidyle, possède un équivalent époxy de 129 g / eq. Plus l'équivalent d'époxy en poids est élevé, plus la teneur en oligomère et/ou mono-glycidyléther d'isosorbide est élevée : on recherchera donc à minimiser cet équivalent d'époxy.

Aucun des documents de l'état de la technique précité qui visent des procédés de préparation d'éthers de bis-anhydrohexitols ne s'intéresse précisément à ce problème complexe qui consiste à augmenter la sélectivité vis-à-vis des dérivés difonctionnels au détriment des dérivés monofonctionnels et des oligomères. Indépendamment de cette considération, l'homme du métier aurait de toute façon éludé un certain nombre de procédés considérés comme non applicables industriellement, car mettant en oeuvre des produits dangereux comme l'hydrure métallique ou le sodium métallique (US 3,272,845), des solvants (WO 2008 147472) ou le trifluorure de bore (WO 2008 147473 dans son exemple 3). D'autre part, les procédés reposant sur des conditions de température et de pression élevées (US 4,770,871) présentent l'inconvénient de mettre en oeuvre des dispositifs couteux et complexes.

L'homme du métier se serait alors tourné vers les méthodes consistant à faire réagir à pression atmosphérique l'isosorbide et l'épichlorhydrine, à ajouter très lentement un réactif basique et à réaliser une distillation azéotropique (US 3,041,300 et WO 2008 147473). EP2301941 et WO2008/147473 décrivent également un tel procédé. Chatti et al. (2001) et Davy-Louis Versace et al. (2013) décrivent des procédés similaires mais conduits sans distillation. Or, la société Demanderesse a démontré dans la partie expérimentale de la présente demande que ces procédés conduisaient à des compositions présentant une teneur trop importante en dérivés monofonctionnels ainsi qu'à une teneur trop importante en oligomères ou un équivalent d'époxy trop élevé.

Poursuivant ses recherches à travers de très nombreux travaux, la société Demanderesse est parvenue à mettre au point un procédé de fabrication de compositions d'éthers de bis-anhydrohexitol, à la fois exempt de solvants et d'autres composés potentiellement dangereux comme l'hydrure métallique, le sodium métallique, ou le trifluorure de bore. De plus, ce procédé conduit à des compositions présentant une richesse très importante en diepoxy d'isosorbide par rapport aux monoepoxy d'isosorbide, et une faible teneur en oligomères ou un équivalent d'époxy avantageusement plus faible que pour les compositions de l'art antérieur.

Le procédé de fabrication d'une composition d'éthers de bis-anhydrohexitols, objet de la présente demande, comprend les étapes suivantes :

a) mettre en contact un dianhydrohexitol et un halogénure organique,

b) placer le mélange ainsi obtenu de dianhydrohexitol et d'halogénure organique sous vide de manière à obtenir une dépression comprise entre 100 mbars et 1000 mbars,

c) chauffer le mélange sous vide à une température comprise entre 50°C et 120°C et réaliser ainsi une distillation azéotropique,

d) ajouter ensuite au dit mélange un réactif basique pendant une durée comprise entre 1 heure et 10 heures et poursuivre alors la distillation azéotropique,

e) récupérer la composition d'éthers de bis-anhydrohexitols après une étape de filtration, concentration du filtrat et éventuellement une étape de purification.

Comme indiqué précédemment, un tel procédé permet l'obtention de compositions présentant une richesse très importante en diépoxy d'isosorbide par rapport aux monoépoxy d'isosorbide, et une faible teneur en oligomères ou un équivalent d'époxy avantageusement plus faible que pour les compositions de l'art antérieur.

Un tel résultat est d'autant plus surprenant que rien dans l'état de la technique ne décrivait ou ne suggérait de mettre en oeuvre, sous une pression réduite, un procédé de synthèse d'éthers de bis-anhydrohexitol. Au contraire, il était indiqué de procéder soit à pression élevée (US 4,770,871) soit à pression atmosphérique (ensemble des autres documents discutés jusqu'ici).

La première étape du procédé selon l'invention (étape a) consiste donc à mettre en contact un dianhydrohexitol et un halogénure organique.

Le dianhydrohexitol est préférentiellement un isohexitol, plus préférentiellement choisi parmi l'isosorbide, l'isomannide et l'isoidide, et est, le plus préférentiellement, l'isosorbide.

L'halogénure organique est préférentiellement choisi parmi l'épibromohydrine, l'épifluorohydrine, l'épiiodohydrine et l'épichlorhydrine, et est, plus préférentiellement, l'épichlorhydrine.

Cet halogénure organique est préférentiellement introduit en excès par rapport aux fonctions hydroxyles du dianhydrohexitol. Ainsi, pour 1 mole de dianhydrohexitol on introduira préférentiellement entre 2 et 10 moles d'halogénure organique et plus préférentiellement environ 5 moles.

Cette première étape de mise en contact d'un dianhydrohexitol et d'un halogénure organique (étape a) est effectuée dans tout dispositif bien connu de l'homme du métier, permettant de réaliser la mise en contact entre 2 réactifs chimiques, et étant muni d'organes de chauffage et d'agitation. Il peut s'agir, à titre d'exemple, d'un réacteur double enveloppe. Le dispositif en question doit également être équipé d'un organe permettant de réaliser un vide partiel et d'un organe permettant de conduire une distillation azéotropique, tel qu'un montage Dean-Stark inversé surmonté d'un réfrigérant.

Après cette première étape de mise en contact (étape a), on réalise ensuite un vide partiel dans le dispositif à l'aide d'une pompe à vide, la dépression correspondante étant comprise entre 100 mbars et 1000 mbars (étape b). Ceci

signifie que la pression dans le milieu réactionnel est égale à la différence entre la pression atmosphérique (1013 mbars) et la pression revendiquée (entre 100 mbars et 1000 mbars), soit une pression comprise entre 13 mbars et 913 mbars.

Selon un mode de réalisation, l'étape b) est réalisée de manière à obtenir une dépression comprise entre 100 et 800 mbars, en particulier entre 100 et 600 mbars.

Au cours de la troisième étape du procédé de l'invention (étape c), on chauffe le mélange entre le dianhydrohexitol et l'halogénure organique à une température comprise entre 50°C et 120°C.

La température du fluide caloporteur circulant dans la double enveloppe du réacteur doit être réglée de manière à être au moins égale à la température d'ébullition de l'halogénure organique utilisé, de manière à commencer la distillation azéotropique. Au cours de cette première phase de distillation, ladite distillation ne concerne que l'halogénure organique : en d'autres termes, seule une partie de l'halogénure organique est éliminée par distillation. Par ailleurs, la température d'ébullition qu'il convient de prendre en compte est la température d'ébullition de l'halogénure organique sous la pression partielle qui règne dans le dispositif.

A titre d'exemple, l'épichlorhydrine présente une température d'ébullition de 116°C à pression atmosphérique, cette température d'ébullition étant environ égale à 80°C sous un vide partiel de 275 mbars. De manière pratique, on se placera à une température légèrement supérieure (environ 3°C de plus) à la température d'ébullition pour l'halogénure organique considéré et pour la dépression imposée.

Au cours de la quatrième étape du procédé de l'invention (étape d), on ajoute alors au mélange diahydrohexitol/halogénure organique un réactif basique, pendant une durée comprise entre 1 heure et 10 heures.

La quantité de réactif basique est préférentiellement la quantité stoechiométrique par rapport au nombre de fonctions hydroxyles du dianhydrohexitol (par exemple : 2 moles de soude pour 1 mole d'isosorbide). On peut néanmoins choisir de se placer en léger excès par rapport à cette stoechiométrie.

Le réactif basique est choisi parmi les hydroxydes de lithium, potassium, calcium et sodium éventuellement sous forme d'une solution aqueuse, et est, très préférentiellement, une solution aqueuse d'hydroxyde de sodium. Dès l'introduction du réactif basique (étape d), il y a formation d'eau par réaction entre le dianhydrohexitol et l'halogénure organique, de même qu'il peut y avoir apport d'eau supplémentaire par introduction du réactif basique sous forme de solution aqueuse. La distillation concerne alors le mélange eau et halogénure organique, le premier étant éliminé et le second retournant dans le milieu réactionnel. Dans le cas d'un dispositif Dean-Stark : l'eau constitue la phase supérieure qui est éliminée, alors que l'halogénure en partie inférieure est retourné dans le milieu réactionnel.

La distillation azéotropique est poursuivie jusqu'à élimination complète de l'eau. Ainsi le milieu réactionnel est encore chauffé pendant une durée comprise entre 30 minutes et 1 heure après la fin de l'addition du réactif basique.

Dans une variante préférentielle, un catalyseur de transfert de phase est ajouté lors de la première étape (étape a). On parvient ainsi à augmenter de manière encore plus sensible la fluidité des produits fabriqués, tout en maintenant une proportion très importante de dérivés diépoxy d'isosorbide par rapport aux dérivés monoépoxy d'isosorbide.

Le catalyseur de transfert de phase est préférentiellement choisi parmi les halogénures, sulfates ou hydrogénosulfates de tétra-alkylammonium et plus préférentiellement parmi le bromure de tétrabutylammonium et l'iodure de tétrabutylammonium. La quantité de catalyseur de transfert de phase est comprise entre 0,01 et 5%, préférentiellement entre 0,1% et 2% plus préférentiellement 1% en poids par rapport au dianhydrohexitol. On parvient alors à réduire très notablement l'indice d'époxy, ce qui signifie que la quantité d'oligomères dans le milieu est largement réduite.

Le milieu réactionnel est enfin filtré afin d'éliminer les sels formés au cours de la réaction entre l'halogénure et le dianhydrohexitol, comme le chlorure de sodium dans le cas de l'épichlorhydrine. Les sels ainsi récupérés sont lavés une nouvelle fois avec de l'épichlorhydrine. Les eaux de lavages sont ajoutées au premier filtrat puis concentrées de manière à éliminer notamment l'épichlorhydrine. L'étape de concentration se fait par exemple par distillation sous vide, par exemple dans un dispositif type rotavapor et/ou évaporateur film raclée. Lors de cette étape de concentration, le produit brut ou composition d'éthers de bis-anhydrohexitols est chauffé progressivement jusqu'à 140°C et la pression est diminuée jusqu'à 1 mbar.

Eventuellement, une étape supplémentaire de purification par distillation sous pression réduite (<1mbar) peut être réalisée au moyen d'un échangeur à surface raclée afin de séparer les oligomères du di-glycidyléther de dianhydrohexitol. Cette étape est distincte de celle décrite dans le paragraphe précédent.

Un autre objet de la présente invention repose sur les compositions susceptibles d'être obtenues selon le procédé de l'invention.

Un dernier objet de la présente invention repose sur l'utilisation de ces compositions pour la fabrication de matériaux composites, de revêtements et d'adhésifs.

Ces compositions peuvent aussi être utilisées pour la synthèse de vinylester par réaction avec des acides (méth)acrylique. Ces monomères photoréticulables (vinylesters) pourront alors être utilisés pour la fabrication de résines dentaires, coque de bateau, revêtement de spécialité.

Les compositions selon l'invention peuvent être utilisées dans des réactions de polycondensation afin d'obtenir un réseau tridimensionnel et un matériau thermorigide.

Dans ce cas, elles peuvent être utilisées seules (réactions d'homopolymérisation) ou en association avec

d'autres monomères (réactions de co-polymérisation).

Parmi les co-monomères on peut citer les autres dérivés époxy mais aussi des agents dit durcisseurs ou de réticulation tels que les amines, polyetheramine, polyamides, amidoamines, bases de Mannich, anhydrides, polyesters polycarboxyliques, les mercaptans, les résines phénoliques, les résines mélamine, urée et phenol-formaldéhyde. Des catalyseurs type acide de Lewis, amine tertiaire, imidazole peuvent également être ajoutés à la formulation afin d'initier et/ou accélérer la réticulation. Les réactions de réticulation se feront à une température allant de 5° à 260°C.

Les matériaux, résines obtenues à partir des compositions d'éthers de bis-anhydrohexitols sont plus résistantes chimiquement et mécaniquement et présentent en outre une température de transition vitreuse (Tg) plus élevée, par rapport aux mêmes matériaux obtenus avec les éthers de bis-anhydrohexitols selon l'art antérieur, comme démontré ci-après.

**EXEMPLES**

**Réactifs :**

Isosorbide : produit Polysorb™ P commercialisé par la société Roquette Frères
Epichlorhydrine : commercialisé par la société Sigma-Aldrich
Bromure de triméthyl ammonium : commercialisé par la société Sigma-Aldrich

**Exemple 1** : Essais selon l'art antérieur.

6 essais (essais 1 à 6) ont été menés au cours desquels on a fait réagir l'isosorbide et l'épichlorhydrine, avec ajout d'une solution aqueuse de soude, la distillation azéotropique étant conduite à pression atmosphérique.

A titre d'exemple, voici comment on a procédé pour l'essai n°1.

On introduit, dans un réacteur double enveloppe de 1 litre, muni d'un bain thermostaté à fluide caloporteur, d'un système d'agitation mécanique à pâle, d'un système de contrôle de la température du milieu réactionnel et d'un Dean Stark inversé surmonté d'un réfrigérant, 125 g d'isosorbide (0,86 mol, 1 équivalent molaire) puis 395,6 g d'épichlorhydrine (4,27 mol, 5 équivalents molaires).

On chauffe alors le mélange réactionnel jusqu'à 116°C (température d'ébullition de l'épichlorhydrine = 116°C à pression atmosphérique) pendant 30 minutes.

On ajoute alors progressivement 136,9 g d'une solution aqueuse d'hydroxyde de sodium à 50 % (1,71 mol, 2 équivalents molaires).

L'addition dure au total 6 h 12 ; la distillation azéotropique se poursuit et l'eau formée par réaction entre l'halogénure et le dianhydrohexitol est éliminée.

On filtre sous vide le milieu réactionnel afin d'en éliminer le chlorure de sodium formé au cours du temps. On lave finalement les sels avec de l'épichlorhydrine qui est ensuite éliminé par évaporation sous pression réduite au rotavapor.

On obtient alors la composition de diglycidyléther d'isosorbide ou contenant majoritairement du diglycidyléther d'isosorbide sous forme d'un liquide limpide (viscosité Brookfield à 25°C de 19800 mPa.s) et légèrement coloré, ayant un équivalent d'époxy de 216 g / équivalent.

Le tableau 1 récapitule les conditions opératoires, et notamment :

- la quantité d'épichlorhydrine mise en oeuvre, exprimée en équivalent molaire d'épichlorhydrine par rapport au nombre de mole de l'isosorbide (Eq Mol EPI)
- la quantité d'hydroxyde de sodium mise en oeuvre, exprimée en équivalent molaire d'hydroxyde de sodium par rapport au nombre de mole de l'isosorbide (Eq Mol NaOH)
- le temps d'introduction de la soude (temps intro NaOH)

Ce tableau indique également la répartition déterminée par Chromatographie en Phase Gazeuse (CPG) (en % de surface) des différents constituants du produit final/ de la composition finale.

Dans tous les exemples de la présente demande, l'analyse CPG est réalisée sur une colonne capillaire de type DB1 (30m x 0,32 mm, épaisseur de film de 0,25μm). La quantification des espèces consiste à calculer la proportion relative des aires des pics du chromatogramme, le % de chaque espèce (x) étant égal à l'aire du pic de l'espèce (x) divisé par la somme de l'aire de tous les pics.

**Exemple 2** : Essais selon l'invention

4 essais selon l'invention (essais 7 à 10) ont été réalisés au cours desquels on a fait réagir l'isosorbide et l'épichlorhydrine, avec ajout d'une solution aqueuse de soude, la distillation azéotropique étant conduite sous un vide partiel.
A titre d'exemple, voici comment on a procédé pour l'essai n°7.
On introduit dans un réacteur double enveloppe de 1 litre, muni d'un bain thermostaté à fluide caloporteur, muni d'un système d'agitation mécanique à pâle, d'un système de contrôle de la température du milieu réactionnel et d'un Dean Stark inversé surmonté d'un réfrigérant, 125 g d'isosorbide (0,86 mol, 1 équivalent molaire) puis 395,6 g d'épichlorhydrine (4,27 mol, 5 équivalents molaires).
Le système est amené à une pression de 568 mbar relatif. On chauffe alors le mélange réactionnel jusqu'à 100°C (température d'ébullition = 100°C à 568 mbar) pendant 30 minutes avant de débuter l'addition contrôlée de 136,9 g d'une solution aqueuse d'hydroxyde de sodium à 50% (1,71 mol, 2 équivalents molaires). L'addition dure au total 3 h 05. L'eau est éliminée en continu par distillation azéotropique.
On filtre sous vide le milieu réactionnel afin d'en éliminer le chlorure de sodium formé au cours du temps. On lave les sels avec de l'épichlorhydrine qui est ensuite éliminé par évaporation sous pression réduite au rotavapor.
On obtient alors la composition de diglycidyléther d'isosorbide ou contenant majoritairement du diglycidyléther d'isosorbide sous forme d'un liquide limpide (viscosité Brookfield à 25°C de 13900 mPa.s) ayant un équivalent d'époxy de 200 g / équivalent.

**Exemple 3** : Essais selon l'invention avec l'emploi d'un catalyseur de transfert de phase

6 autres essais selon l'invention (essais 11 à 16) ont été réalisés au cours desquels où on a fait réagir l'isosorbide et l'épichlorhydrine en présence d'un catalyseur de transfert de phase, avec ajout d'une solution aqueuse de soude, la distillation azéotropique étant conduite sous un vide partiel.
A titre d'exemple, voici comment on a procédé pour l'essai n°11.
On introduit dans un réacteur double enveloppe de 1 litre, chauffé par un bain thermostaté à fluide caloporteur, muni d'un système d'agitation mécanique à pâle, d'un système de contrôle de la température du milieu réactionnel et d'un Dean Stark inversé surmonté d'un réfrigérant, 125 g d'isosorbide (0,86 mol, 1 équivalent molaire), 395,6 g d'épichlorhydrine (4,27 mol, 5 équivalents molaires) puis 1,25 g de bromure de triéthylammonium (1% massique par rapport à l'isosorbide).
Le système est amené à une pression de 275 mbar relatif. On chauffe le mélange réactionnel jusqu'à 80°C (Température d'ébullition = 80°C à 275 mbar) avant de débuter l'addition contrôlée de 136,9 g d'une solution aqueuse d'hydroxyde de sodium à 50% (1,71 mol, 2 équivalents molaires). L'addition dure au total 2 h 50. L'eau est alors éliminée en continu par distillation azéotropique.
On filtre sous vide le milieu réactionnel afin d'en éliminer le chlorure de sodium formé au cours du temps et le catalyseur. On lave les sels à l'aide d'épichlorhydrine qui est ensuite éliminé par évaporation sous pression réduite au rotavapor.
On obtient alors la composition de diglycidyléther d'isosorbide ou contenant majoritairement du diglycidyléther d'isosorbide sous forme d'un liquide limpide (viscosité Brookfield à 25°C de 4350 mPa.s) ayant un équivalent d'époxy de 176 g / équivalent.
Le tableau 2 récapitule les conditions opératoires employées pour les essais 7 à 16, et notamment :

- la quantité d'épichlorhydrine mise en oeuvre, exprimée en équivalent molaire d'épichlorhydrine par rapport au nombre de mole de l'isosorbide (Eq Mol EPI)
- la quantité d'hydroxyde de sodium mise en oeuvre, exprimée en équivalent molaire d'hydroxyde de sodium par rapport au nombre de mole de l'isosorbide (Eq Mol NaOH)
- le temps d'introduction de la soude (temps intro NaOH)
- la dépression (mBars)
- la nature du catalyseur

Ce tableau indique également la répartition déterminée par CPG (en % de surface) des différents constituants du produit final.
La comparaison entre le tableau 1 et le tableau 2 démontre que le procédé selon l'invention permet d'obtenir des compositions dans lesquelles la proportion de diglycidyléther d'isosorbide par rapport au monoglycidylether d'isosorbide est considérablement plus importante et l'équivalent d'époxy donc la teneur en oligomères produits parasites plus faible.
Enfin, la variante préférée de l'invention consistant à mettre en oeuvre un catalyseur de transfert de phase,

permet d'exacerber ces effets. Les essais n° 11 à 15 qui mettent en oeuvre 1 % en poids de catalyseur permettent notamment de diminuer de manière très conséquente l'équivalent epoxy, et donc de faire chuter la teneur en oligomères.

**Tableau 1**

| Essai n° | 1 | 2 | 3 | 4 | 5 | 6 |
|---|---|---|---|---|---|---|
| Eq Mol EPI | 5 | 5 | 5 | 10 | 10 | 10 |
| Eq Mol NaOH | 2 | 2 | 2 | 2 | 2 | 2 |
| Temps intro NaOH | 6h12 | 3h06 | 1h04 | 9h37 | 6h12 | 3h08 |
| isosorbide | 0,4% | 0,1% | 0,0% | 1,0% | 0,3% | 0,1% |
| monoglycidylether isosorbide | 18,7% | 13,4% | 14,1% | 33,7% | 28,7% | 28,5% |
| diglycidylether isosorbide | 40,8% | 49,2% | 44,7% | 30,8% | 38,0% | 40,9% |
| monoglycidylether di-isosorbide | 4,5% | 3,4% | 4,2% | 6,1% | 5,0% | 4,6% |
| diglycidylether di-isosorbide | 10,8% | 14,9% | 15,6% | 9,0% | 10,1% | 10,3% |
| glycerol | 0,5% | 0,3% | 0,6% | 0,6% | 0,3% | 0,2% |
| autres | 24,3% | 18,7% | 20,8% | 18,8% | 17,6% | 15,4% |
| % diglycidylether isosorbide/ (mono+diglycidylether isosorbide) | 68,6% | 78,5% | 76,0% | 47,8% | 57,0% | 58,9% |
| Equivalent Epoxy (g/eq) | 215 | 216 | 216 | 234 | 224 | 224 |

**Tableau 2**

| Essai n° | 7 | 8 | 9 | 10 | 11 | 12 | 13 | 14 | 15 | 16 |
|---|---|---|---|---|---|---|---|---|---|---|
| Eq Mol EPI | 5 | 10 | 5 | 5 | 5 | 5 | 10 | 5 | 5 | 5 |
| Eq Mol NaOH | 2 | 2 | 2 | 2 | 2 | 2 | 2 | 2 | 2 | 2 |
| Temps intro NaOH | 3h05 | 3h09 | 4h30 | 2h56 | 2h50 | 3h00 | 2h54 | 3h02 | 3h04 | 2h55 |
| Pression (m Bar) | 568 | 275 | 275 | 192 | 275 | 275 | 275 | 275 | 275 | 275 |
| Catalyseur | - | - | - | - | TEAB | TEAB | TEAB | TEAC | TBAI | TEAB* |
| isosorbide | 0,1% | 0,1% | 0,0% | 0,2% | 0,0% | <0.1% | 0,0% | <0.1% | 0,0% | 0,0% |
| monoglycidylether isosorbide | 12,6% | 8,6% | 4,2% | 4,8% | 5,1% | 10,1% | 11,7% | 10,4% | 6,4% | 4,5% |
| diglycidylether isosorbide | 57,0% | 60,7% | 65,8% | 58,1% | 63,0% | 63,9% | 66,3% | 56,0% | 64,2% | 61,6% |
| monoglycidylether di-isosorbide | 2,4% | 1,1% | 0,6% | 0,8% | 1,0% | 1,7% | 1,0% | 2,3% | 1,0% | 0,9% |
| diglycidylether di-isosorbide | 14,5% | 12,6% | 13,1% | 15,8% | 12,5% | 12,4% | 9,3% | 14,4% | 13,4% | 15,4% |
| glycerol | 0,2% | <0.1% | 0,1% | 0,2% | 0,0% | <0.1% | 0,0% | 0,1% | <0.1% | 0,0% |
| autres | 13,2% | 16,8% | 16,2% | 20,1% | 18,4% | 11,7% | 11,7% | 16,7% | 14,9% | 17,6% |
| Diglycidylether isosorbide /(mono+diglycidylet her isosorbide) | 81,9% | 87,6% | 94,0% | 92,4% | 92,5% | 86,4% | 85,0% | 84,3% | 90,9% | 93,2% |
| Equivalent Epoxy (g/eq) | 200 | 203 | 211 | 209 | 176 | 178 | 179 | 187 | 181 | 195 |

* les essais 11 à 15 sont réalisés avec 1% en poids d'agent de transfert alors que l'essai 16 ne met en oeuvre que 0,1 % de cet agent
TEAB : Triethyl ammonium bromide
TEAC : Triethyl ammonium chloride
TBAI : Triethyl ammonium iodide

**Exemple 4** : Réalisation de résines à partir de compositions selon l'invention ou selon l'art antérieur.

Des résines époxy à partir des compositions de glycidyléthers d'isosorbide et en présence d'un durcisseur de type amine (l'isophorone diamine) ont été préparées.

La quantité d'isophorone diamine introduite est calculée de manière à ce que le rapport du nombre de groupement -NH sur le nombre de groupement époxy soit égal à 1. L'isophorone diamine est disponible sous la marque Vestamid® IPD par Evonik. L'équivalent de groupement -NH en poids est de 42,5g/eq. La formule utilisée pour calculer les mises en oeuvre de diamine est la suivante :

$$m\,(isophorone\ diamine) = \frac{mepoxy\ \times 42{,}5}{Equivalent\ epoxy}$$

A titre d'exemple, voici comment on a procédé pour l'essai 17.
10,630 g du produit obtenu à l'essai n°2 sont mélangés à température ambiante avec 1,934 g d'isophorone diamine pendant 1 minute. Le mélange, homogène et s'écoulant à température ambiante, est placé dans un moule en silicone (L=43mm, l=20mm). La réticulation est effectuée en étuve pendant 1 heure à 80°C et 2h à 180°C.

On obtient alors un matériau solide à température ambiante et ayant une température de transition vitreuse (Tg) de 66°C. La température de transition vitreuse est mesurée par DSC au deuxième passage d'une rampe de température de -100 à 200°C à 10°C/min.

Le tableau 3 récapitule les résultats obtenus suivant les compositions de glycidyléthers d'isosorbide utilisées.

**Tableau 3**

| Essai n° | 17 | 18 | 19 | 20 |
|---|---|---|---|---|
| Ref de l'essai correspondant au glycidyléther d'isosorbide utilisé | 4 (hors invention) | 5 (hors invention) | 13 (selon l'invention) | 15 (selon l'invention) |
| Equivalent d'époxy (g/eq) | 234 | 224 | 179 | 181 |
| Tg (°C) | 66 | 75,2 | 94,5 | 99,4 |

On note une très nette augmentation de la température de transition vitreuse pour les essais 19 et 20, réalisés avec les compositions selon l'invention, par rapport aux essais 17 et 18 qui mettent en oeuvre des compositions selon l'art antérieur.

## Revendications

**1.** Procédé de fabrication de compositions d'éthers de bis-anhydrohexitols, comprenant les étapes suivantes:

a) mettre en contact un dianhydrohexitol et un halogénure organique,
b) placer le mélange ainsi obtenu de dianhydrohexitol et d'halogénure organique sous vide de manière à obtenir une dépression comprise entre 100 mbars et 1000 mbars,
c) chauffer le mélange sous vide à une température comprise entre 50°C et 120°C et réaliser ainsi une distillation azéotropique,
d) ajouter ensuite au dit mélange un réactif basique pendant une durée comprise entre 1 heure et 10 heures et poursuivre alors la distillation azéotropique,
e) récupérer la composition d'éthers de bis-anhydrohexitols après une étape de filtration, concentration du filtrat et éventuellement une étape de purification.

**2.** Procédé selon la revendication 1, **caractérisé en ce que** le dianhydrohexitol est un isohexitol, plus préférentiellement choisi parmi l'isosorbide, l'isomannide et l'isoidide, et est, le plus préférentiellement, l'isosorbide.

**3.** Procédé selon l'une quelconque des revendications précédentes, **caractérisé en ce que** l'halogénure organique est choisi parmi l'épibromohydrine, l'épifluorohydrine, l'épiiodohydrine et l'épichlorhydrine, et est, plus préférentiellement, l'épichlorhydrine.

4. Procédé selon l'une quelconque des revendications précédentes, **caractérisé en ce que** le réactif basique est choisi parmi les hydroxydes de lithium, potassium, calcium et sodium éventuellement sous forme d'une solution aqueuse, et est très préférentiellement une solution aqueuse d'hydroxyde de sodium.

5. Procédé selon l'une quelconque des revendications précédentes, **caractérisé en ce qu'**un catalyseur de transfert de phase est ajouté lors de l'étape a).

6. Procédé selon la revendication 5, **caractérisé en ce que** le catalyseur de transfert de phase est choisi parmi les halogénures, sulfates ou hydrogénosulfates de tétra-alkylammonium et plus préférentiellement parmi le bromure de tétrabutylammonium et l'iodure de tétrabutylammonium.

7. Procédé selon l'une quelconque des revendications 5 ou 6, caractérisé en ce la quantité de catalyseur de transfert de phase est comprise entre 0,01 et 5%, préférentiellement entre 0,1% et 2% plus préférentiellement 1% en poids par rapport au dianhydrohexitol.

## Patentansprüche

1. Verfahren zur Herstellung von bis-Anhydrohexitol-Ether-Zusammensetzungen, welches die folgenden Schritte umfasst:

   a) Inkontaktbringen eines Dianhydrohexitols und eines organischen Halogenids,
   b) Platzieren der so erhaltenen Mischung aus Dianhydrohexitol und organischem Halogenid im Vakuum zum Erhalten eines Unterdrucks von 100 mbar bis 1000 mbar,
   c) Erhitzen der Mischung im Vakuum bei einer Temperatur von 50 °C bis 120 °C und auf diese Weise Durchführen einer azeotropischen Destillation,
   d) Hinzufügen eines basischen Reaktionsmittels zu der Mischung während eines Zeitraums von 1 h bis 10 h und Weiterführen der azeotropischen Destillation,
   e) Gewinnen der bis-Anhydrohexitol-Ether-Zusammensetzung nach einem Filtrationsschritt, Konzentration des Filtrats und ggf. einen Reinigungsschritt.

2. Verfahren nach Anspruch 1, **dadurch gekennzeichnet, dass** das Dianhydrohexitol ein Isohexitol ist, stärker bevorzugt gewählt aus Isosorbid, Isomannid und Isoidid, und am stärksten bevorzugt ein Isosorbid ist.

3. Verfahren nach einem der vorhergehenden Ansprüche, **dadurch gekennzeichnet, dass** das organische Halogenid gewählt ist aus Epibromhydrin, Epifluorhydrin, Epiiodhydrin und stärker bevorzugt Epichlorhydrin ist.

4. Verfahren nach einem der vorhergehenden Ansprüche, **dadurch gekennzeichnet, dass** das basische Reaktionsmittel gewählt ist aus Lithium-, Kalium-, Calcium- und Natriumhydroxiden, ggf. als wässrige Lösung, und stark bevorzugt Natriumhydroxid in wässriger Lösung ist.

5. Verfahren nach einem der vorhergehenden Ansprüche, **dadurch gekennzeichnet, dass** in Schritt a) ein Phasentransferkatalysator hinzugefügt wird.

6. Verfahren nach Anspruch 5, **dadurch gekennzeichnet, dass** der Phasentransferkatalysator gewählt ist aus Halogeniden, Tetraalkylammonium-Sulfaten oder-Hydrogensulfaten und stärker bevorzugt aus Tetrabutylammoniumbromid und Tetrabutylammoniumiodid.

7. Verfahren nach einem der Ansprüche 5 oder 6, **dadurch gekennzeichnet, dass** die Menge Phasentransferkatalysator im Verhältnis zum Dianhydrohexitol zwischen 0,01 und 5 Gewichtsprozent, bevorzugt zwischen 0,1 und 2 Gewichtsprozent, stärker bevorzugt bei 1 Gewichtsprozent liegt.

## Claims

1. A process for producing bis-anhydrohexitol ether compositions, comprising the following steps:

   a) bringing a dianhydrohexitol into contact with an organic halide,

b) placing the resulting mixture of dianhydrohexitol and organic halide under vacuum so as to obtain a negative pressure of between 100 mbar and 1000 mbar,
c) heating the mixture under vacuum at a temperature of between 50°C and 120°C and thus carrying out an azeotropic distillation,
d) then adding to said mixture a basic reagent for a period of between 1 hour and 10 hours and then continuing the azeotropic distillation,
e) recovering the bis-anhydrohexitol ether composition after a filtration step, concentration of the filtering and optionally a purification step.

**2.** The process as claimed in claim 1, **characterized in that** the dianhydrohexitol is an isohexitol, more preferentially chosen from isosorbide, isomannide and isoidide, and is, more preferentially, isosorbide.

**3.** The process as claimed in either one of the preceding claims, **characterized in that** the organic halide is chosen from epibromohydrin, epifluorohydrin, epiiodohydrin and epichlorohydrin, and is, more preferentially, epichlorohydrin.

**4.** The process as claimed in any one of the preceding claims, **characterized in that** the basic reagent is chosen from lithium hydroxide, potassium hydroxide, calcium hydroxide and sodium hydroxide optionally in the form of an aqueous solution, and is very preferentially an aqueous solution of sodium hydroxide.

**5.** The process as claimed in any one of the preceding claims, **characterized in that** a phase-transfer catalyst is added during step a).

**6.** The process as claimed in claim 5, **characterized in that** the phase-transfer catalyst is chosen from tetraalkylammonium halides, sulfates or hydrogen sulfates and more preferentially from tetrabutylammonium bromide and tetrabutylammonium iodide.

**7.** The process as claimed in either one of claims 5 and 6, **characterized in that** the amount of phase-transfer catalyst is between 0.01% and 5%, preferentially between 0.1% and 2%, more preferentially 1% by weight relative to the dianhydrohexitol.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 3272845 A
- US 4770871 A
- WO 2008147472 A
- US 3041300 A
- WO 2012157832 A
- WO 2008147473 A
- EP 2301941 A


### Littérature non-brevet citée dans la description


- Détermination of Bisphenol A diglycidyl ether and its hydrolysis products in canned oily foods from the Austrian market. *Z. Lebensm. Unters. Forsch. A,* 1999, vol. 208, 208-211